(19) Europäisches Patentamt
European Patent Office
Office européen des brevets

(11) Publication number: 0 331 899 B1

# (12) EUROPEAN PATENT SPECIFICATION

(45) Date of publication of patent specification: 27.04.94

(51) Int. Cl.[5]: C07D 213/80, C07D 215/54, C07D 221/04

(21) Application number: 89101766.7

(22) Date of filing: 02.02.89

## (54) A method for the sequential oxidation of substituted-8-hydroxy-quinolines to produce substituted-pyridine-2,3-dicarboxylic acids.

(30) Priority: 10.03.88 US 166359

(43) Date of publication of application: 13.09.89 Bulletin 89/37

(45) Publication of the grant of the patent: 27.04.94 Bulletin 94/17

(84) Designated Contracting States: AT BE CH DE ES FR GB GR IT LI LU NL SE

(56) References cited: EP-A- 0 259 687

(73) Proprietor: AMERICAN CYANAMID COMPANY
One Cyanamid Plaza
Wayne, NJ 07470-8426(US)

(72) Inventor: Pascavage, John Joseph
804 West 15th Street
Tyrone Pennsylvania 16686(US)

(74) Representative: Wächtershäuser, Günter, Dr.
Tal 29
D-80331 München (DE)

EP 0 331 899 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention herein described relates to a method for the preparation of substituted pyridine-2,2-dicarboxylic acids via a sequential two part oxidation of the appropriately substituted-8-hydroxyquinoline precursors under basic conditions. European Patent Application No. EP-A-259,687 describes a novel method for the preparation of substituted and unsubstituted pyridine-2,3-dicarboxylic acids using hydrogen peroxide under basic conditions. The novel use of hydrogen peroxide in the presence of base to yield substituted-2,3-pyridinedicarboxylic acids in high purity is a distinct advantage over the known methods for preparing said dicarboxylic acids.

It has now been found that the sequential addition of hypochlorite to the hydrogen peroxide-base oxidation surprisingly decreases the amounts of hydrogen peroxide needed for optimum product purity and significantly increases the product yield. The substituted pyridine-2,3-dicarboxylic acids, so produced, are useful as intermediates in the preparation of novel pyridine and quinoline imidazolinone herbicidal agents. The appropriately substituted pyridine and quinoline 2,3-dicarboxylic anhydrides used as starting materials may be prepared according to the process described in U.S. Patent 4,562,257 from their pyridine and quinoline 2,3-dicarboxylic acid precursors. The sequence of reactions used to obtain herbicidal agents of formula IV from substituted pyridine 2,3-dicarboxylic acids of formula I is illustrated as Flow Diagram I.

FLOW DIAGRAM I

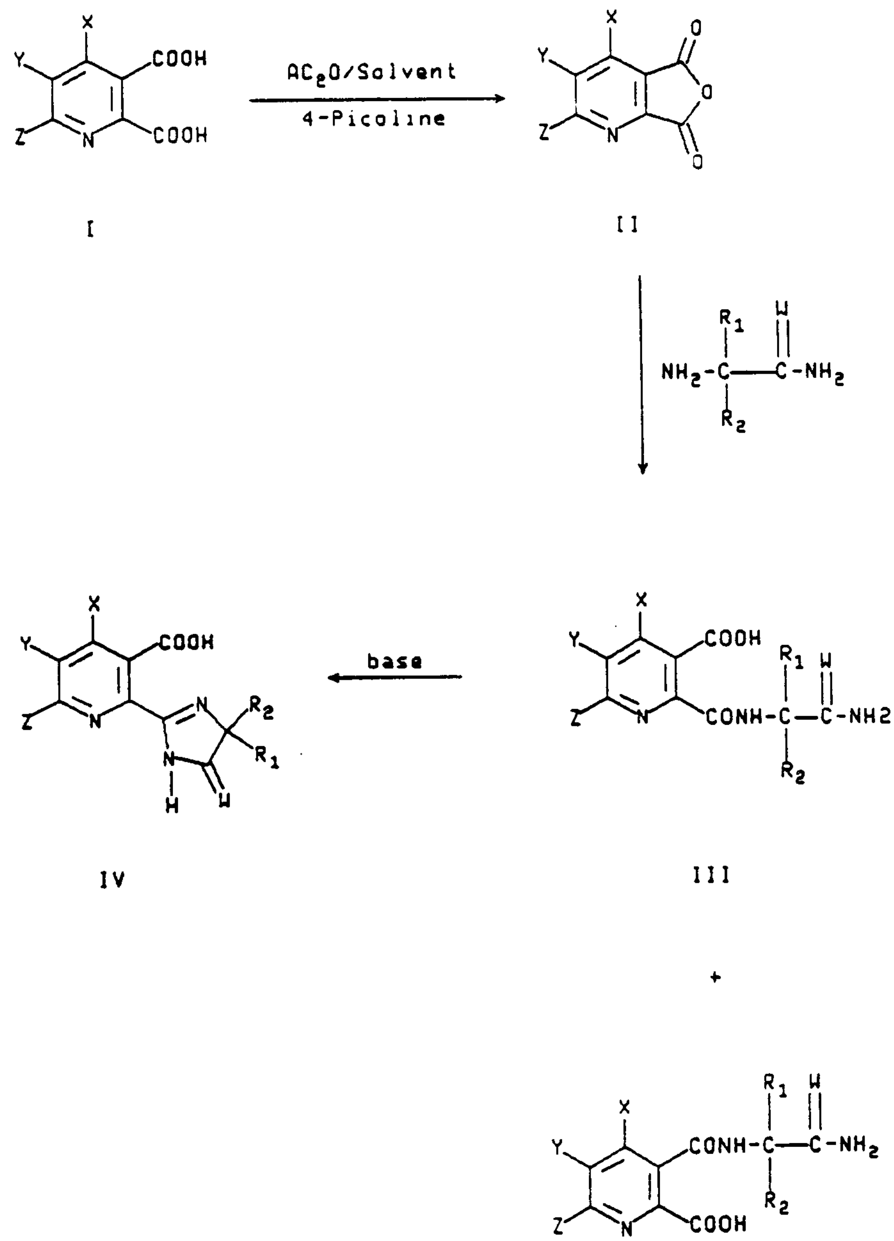


I

II

IV

III

+

## DETAILED DESCRIPTION OF THE INVENTION

The invention relates to a novel method for the preparation of substituted pyridine-2,3-dicarboxylic acids of formula I

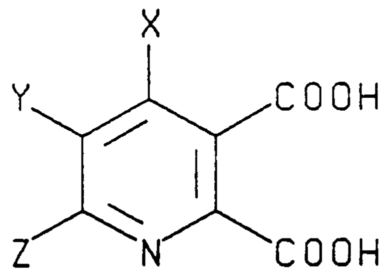

( I )

wherein

X is hydrogen, halogen, or methyl, with the proviso that when Y and Z are taken together to form a ring and YZ is represented by the structure: $-(CH_2)_n-$, where n is 3 or 4, X is hydrogen;

Y and Z each represent members selected from the group consisting of hydrogen, halogen, $C_1$-$C_6$ alkyl, $C_1$-$C_6$ hydroxyalkyl, $C_1$-$C_6$ haloalkyl, $C_1$-$C_6$ aminoalkyl, $C_1$-$C_6$ sulfonylalkyl, nitro, hydroxy, formyl, carboxy, acyl, amido, amino, $C_1$-$C_4$ alkylamino, diloweralkylamino, $C_1$-$C_4$ alkylsulfonyl, sulfonamido, or phenyl optionally substituted with one $C_1$-$C_4$ alkyl group, $C_1$-$C_4$ alkylsulfonyl group, halogen, hydroxy, or trifluoromethyl group; and, when taken together, Y and Z may form a ring in which YZ are represented by the structure: $-(CH_2)_n-$, where n is an integer selected from 3 or 4, provided that X is hydrogen; or

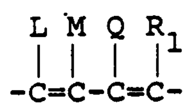

where L, M, Q, and $R_1$ each represent members selected from the group consisting of hydrogen, halogen, $C_1$-$C_4$ alkyl, $C_1$-$C_4$ alkylsulfonyl, $C_1$-$C_4$ haloalkyl, amino, $C_1$-$C_4$ alkylamino, diloweralkylamino, and trifluoromethyl, with the proviso that only one of L, M, Q or $R_1$ may represent a substituent other than hydrogen, halogen, or $C_1$-$C_4$ alkyl.

Compounds of formula I are prepared by oxidizing a substituted hydroxyquinoline of formula II

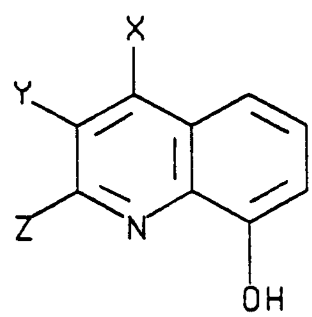

( I I )

wherein X, Y, and Z are as described for formula I in the presence of aqueous base using hydrogen peroxide followed by addition of hypochlorite.

Aqueous bases suitable for use in the method of the invention include alkali metal and alkaline earth metal hydroxides and carbonates such as sodium, potassium, lithium, and calcium hydroxides or carbonates and mixtures thereof. Aqueous sodium hydroxide and aqueous potassium hydroxide are the preferred bases.

In the presence of aqueous base (preferably 5.5 molar equivalents), 8-hydroxyquinolines of formula II are treated with from about 7 to 14 molar equivalents of hydrogen peroxide, but preferably 8 molar equivalents, at 60°-100°C, preferably 85°-90°C. Following the addition, the reaction is held for about one hour, then mineral acid is added to the reaction solution to obtain a pH of about 8-13 (the preferred range being about 10.5-11.5) and the temperature is adjusted to about 25°-90°C, but preferably about 65°-70°C. At this time 1.0-2.0 molar equivalents (but preferably 1.5 molar equivalents) of hypochlorite anion is added as a 5%-30% aqueous solution or is generated in situ by the direct addition of chlorine gas. Reaction temperatures of from 25° to 85°C are suitable, but additional reaction time is necessary at lower temperatures for complete oxidation to occur.

The oxidation of 8-hydroxyquinolines of formula II to pyridine-2,3-dicarboxylic acids of formula I according to the method of this invention is treated as a two part process. The initial step of the oxidation reaction is the cleavage of the nonheteroaromatic ring bearing the hydroxy group by hydrogen peroxide in the presence of aqueous base to give intermediates of formula Ia. The second part of the process is the oxidation of the side chains of formula Ia intermediates of carboxylic acid functional groups via the introduction of hypochlorite anions as illustrated in flow diagram II.

FLOW DIAGRAM II

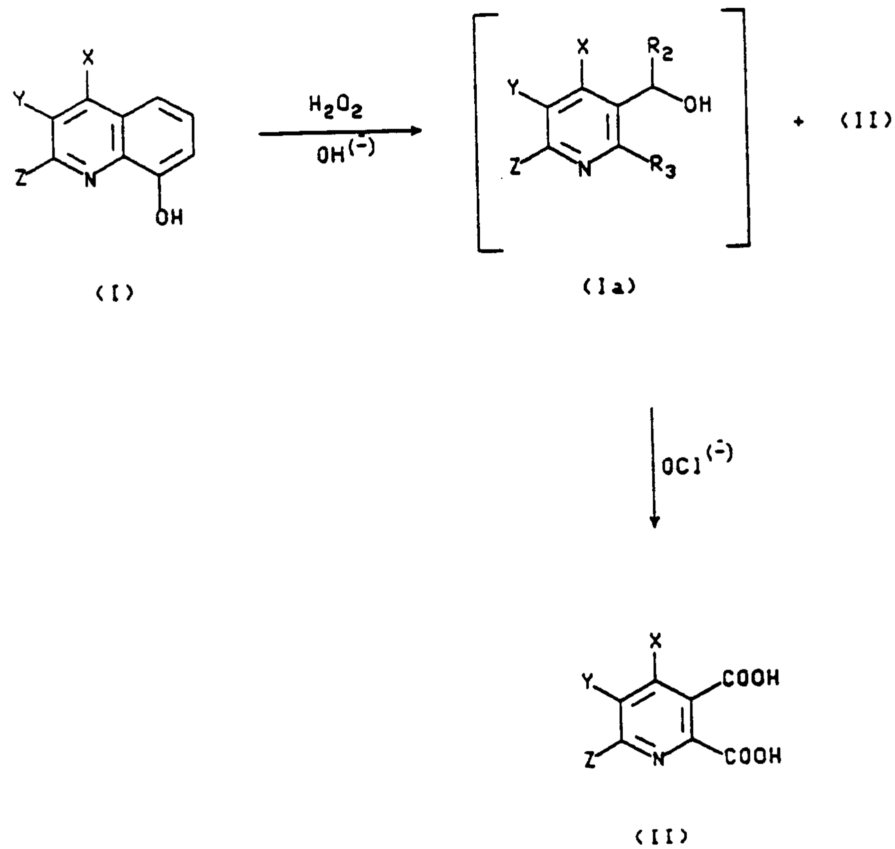


(I) (Ia) (II)

wherein $R_2$ and $R_3$ represent a mixture of members selected from the functional groups consisting of carboxylic acids, glycolic acids, aldehydes, hydroxymethyl groups, and other alkyl groups at intermediate stages of oxidation. It has been found that whereas hydrogen peroxide is preferable for the first oxidation step, i.e. cleavage of the aromatic ring system, surprisingly, hypochlorite anion is preferable for the completion of oxidation of the resulting intermediates to the final dicarboxylic acid products.

The pH of the reaction solution at the time of the introduction of the hypochlorite anion has a great influence on the reaction yield. It has been found that adjustment of the reaction pH to a range of about 10.5-11.5 gives an excellent yield of reaction product.

After the addition of hypochlorite as a 5%-30% aqueous solution or generated in situ by the addition of chlorine gas, the reaction is followed by using a potassium iodide-starch indicator test for the presence of

hypochlorite anions. When the potassium iodide-starch test is negative (usually after one hour), the product dicarboxylic acid can be obtained by acidification of the reaction mixture with a mineral acid and isolated by standard procedures such as filtration or extraction into an appropriate organic solvent.

Among the compounds that can be prepared by this process are those shown below in Table I.

Table I

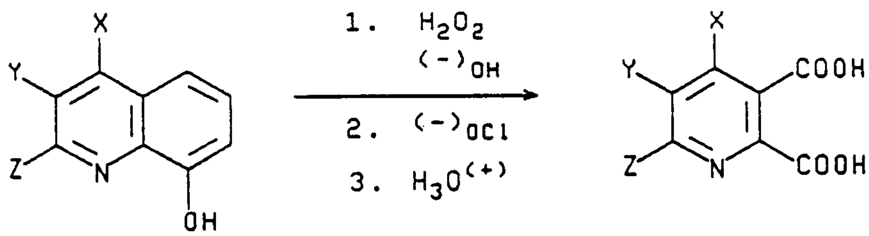


(I)

| X | Y | Z |
|---|---|---|
| H | $CH_3$ | H |
| H | $C_2H_5$ | H |
| $C_2H_5$ | $C_2H_5$ | H |
| H | H | $CH_3$ |
| $CH_3$ | H | H |
| H | $C_2H_5$ | $C_2H_5$ |
| H | $H_2N-C_6-H_{12}$ | H |
| H | Br | H |
| H | $NH_2$ | H |
| H | $NH_2$ | $CH_3$ |
| H | $HOCH_2$ | H |
| H | $CH_2=CH-CH_2$ | $CH_3$ |
| H | $CH_2=CH-CH_2$ | H |
| H | $-SO_2NH_2$ | $CH_3$ |
| H | $CH_3-CH(OH)$ | H |

Formula II starting 8-hydroxyquinolines may readily be prepared by procedures known in the art such as the Skraup reaction, Doebner-Miller reaction or the sulfonation of quinoline.

In order to facilitate a further understanding of the invention, the following examples are presented primarily for the purpose of illustrating more specific details thereof. The invention is not to be limited thereby except as defined in the claims. Unless otherwise noted, all parts are by weight and all degrees are degree centigrade.

## EXAMPLE 1

### Preparation of 5-ethylpyridine-2,3-dicarboxylic acid via hydrogen peroxide and sodium hypochlorite

A stirred mixture of 50% sodium hydroxide (46.2 g, 0.578 mole) and 3-ethyl-8-hydroxyquinoline (50.0 g, 0.289 mol) in 147 mL of water is heated to 90° and treated simultaneously with 5.4 molar equivalents of 35% hydrogen peroxide (152.0 g, 1.56 mole) and 50% sodium hydroxide (81.0 g, 1.01 mole). Another 1.6 molar equivalents of 35% $H_2O_2$ (45.0 g, 0.46 mole) is added. The reaction temperature is maintained at 90-95° until exotherm has subsided, then held at 90-95° for one additional hour. After cooling to 70-80°, the pH of the reaction solution is adjusted to 11.5 using 93% sulfuric acid and 14.3% sodium hypochlorite (108.2 g, 0.207 mole) is added over a one hour period. The reaction is stirred at ambient temperature for 16 hours then filtered. The filtrate is treated with sodium sulfate (50.0 g) and tetrahydrofuran (1066 g). The resulting mixture is stirred and treated with 93% sulfuric acid (75.4 g) to pH 2. The phases are separated. The aqueous phase is extracted with three 130 g portions of tetrahydrofuran. The organic phases are combined and evaporated to dryness in vacuo. The resulting white solid residue is dried at 60° in vacuo for 72 hours, to yield 54.1 g (87.0%, 90.4% purity) of product.

## EXAMPLE 2

### Preparation of 5-methylpyridine-2,3-dicarboxylic acid via hydrogen peroxide and sodium hypochlorite

A stirred mixture of 50% sodium hydroxide (52.0 g, 0.65 mole) and 3-methyl-8-hydroxyquinoline hydrochloride (19.6 g, 0.10 mole) in 153 mL water is heated to 80° and treated with 30% hydrogen peroxide (159.0 g, 14 mole) over a two hour period. The reaction temperature is maintained at 80-85° during the addition, then held at 80-85° for one additional hour. After cooling to 65° (pH 11.6), the reaction solution is treated with 15% sodium hypochlorite (50.0 g, 0.10 mole) and heated at 70° for one hour. After cooling the reaction to 40°, the pH is adjusted to 1.7 with 37% hydrochloric acid (4.5 mL) and is further cooled at 20° for one hour. The reaction mixture is filtered and the filter cake is dried at 60° in vacuo to provide 10.8 g of product as an off-white solid.

## EXAMPLE 3

### Evaluation of the hydrogen peroxide-hypochlorite sequential oxidation of substituted 8-hydroxyquinoline to substituted pyridine 2,3-dicarboxylic acids

In the presence of 5.5 moles of aqueous base, a solution of 3-ethyl-8-hydroxyquinoline was treated at 85-90°, with from 8-14 molar equivalents of hydrogen peroxide. After holding one hour at 85-90° the reaction solution was either cooled to room temperature and the 5-ethylpyridine-2,3-dicarboxylic acid products isolated by standard procedures, or the reaction temperature was adjusted to 65-70°, mineral acid was added to obtain a pH of 10.5-11.5 and 1.0-2.0 molar equivalents of 15% aqueous sodium hypochlorite was added and the 5-ethylpyridine 2,3-dicarboxylic acid product was isolated as above after holding at 65-70° for 1-1 1/2 hours.

The product yields were determined for all experiments and are shown on Table II.

TABLE II

| Oxidation of 3-Ethyl-8-hydroxyquinoline | | | |
|---|---|---|---|
| Experiment Number | Molar Equivalents of $H_2O_2$ | Molar Equivalents of NaOCl | % Yield of 5-Ethylpyridine-2,3-dicarboxylic Acid |
| 1 | 8.0 | 0.0 | 72.0 |
| 1 | 8.0 | 2.0 | 90.8 |
| 1 | 14.0 | 0.0 | 82.8 |
| 2 | 8.0 | 0.0 | 59.3 |
| 2 | 8.0 | 2.0 | 75.3 |
| 2 | 14.0 | 0.0 | 61.0 |
| 3 | 8.0 | 0.0 | 66.0 |
| 3 | 8.0 | 2.0 | 86.2 |
| 3 | 14.0 | 0.0 | 69.1 |
| 4 | 8.0 | 2.0 | 89.6 |
| 4 | 14.0 | 0.0 | 63.6 |
| 5 | 14.0 | 0.0 | 77.0 |
| 5 | 14.0 | 2.0 | 88.0 |
| 6 | 14.0 | 0.0 | 73.0 |
| 6 | 14.0 | 2.0 | 86.0 |
| 7 | 14.0 | 0.0 | 70.5 |
| 7 | 14.0 | 1.0 | 86.2 |

## EXAMPLE 4

Comparison of the sequential hydrogen peroxide-hypochlorite oxidation to hydrogen peroxide alone

After the addition of hydrogen peroxide to a solution of 3-ethyl-8-hydroxyquinoline in 5.5 moles of base at 85-95°, the reaction solution was split. One portion was cooled to room temperature and the 5-ethylpyridine-2,3-dicarboxylicacid product was isolated using standard procedures and the reaction yield was determined. The remaining portion of the reactive solution was treated with mineral acid to a pH of 10-11 at 75-78° then 1.0-2.0 molar equivalents and hypochlorite was added at a 15% aqueous solution. After one hour, 5-ethylpyridine-2,3-dicarboxylic acid was isolated using standard procedures and the reaction yield was determined. These yields are compared in the following table.

TABLE III

| Improvement Of Yields Of 5-Ethylpyridine-2,3-dicarboxylic Acid Via The Oxidations Of 3-Ethyl-8-hydroxyquinoline | | | | |
|---|---|---|---|---|
| Molar Equivalents of $H_2O_2$ | Molar Equivalents 15% NaOCl | pH | Temp. °C | Increase of % Yield |
| 8.0 | 2.0 | 11.0 | 75 | 27.0 |
| 8.0 | 1.0 | - | - | 13.7 |
| 8.0 | 2.0 | 11.0 | 78 | 30.8 |
| 7.0 | 1.5 | 11.0 | 78 | 19.3 |
| 8.0 | 1.5 | 11.0 | 78 | 23.7 |

EXAMPLE 5

Effect of the addition of hypochlorite to the hydrogen peroxide oxidation of 3-ethyl-8-hydroxyquinoline

A solution of 3-ethyl-8-hydroxyquinoline in 5.5 moles of aqueous base was treated with 14.0 molar equivalents of hydrogen peroxide at 85-90°. After one hour, the reaction solution was split and one portion was cooled to room temperature. Using standard procedures, the 5-ethylpyridine-2,3-dicarboxylic acid product was isolated and the product yield was determined. The remaining portion was treated with a 5% aqueous sodium hypochlorite solution and after 16 hours the 5-ethylpyridine-2,3-dicarboxylic acid product was isolated and the reaction yield was determined. These yields are compared in the following table.

TABLE IV

| Improvement Of Yields Of 5-Ethylpyridine-2,3-dicarboxylic Acid As A Result Of The Sequential Addition Of Hypochlorite To A Hydrogen Peroxide Oxidation Of 3-Ethyl-8-hydroxyquinoline | | | | |
|---|---|---|---|---|
| Molar Equivalents of $H_2O_2$ | Molar Equivalents of 5% NaOCl | Temp. | Time Hours | Increase of % yield |
| 14 | 0.25 | ambient | 16 | 2.2 |
| 14 | 0.50 | ambient | 16 | 4.1 |
| 14 | 1.00 | ambient | 16 | 25.7 |
| 14 | 1.50 | ambient | 16 | 25.2 |
| 14 | 2.00 | ambient | 16 | 18.1 |

EXAMPLE 6

Preparation of 3-ethyl-8-hydroxyquinoline

A stirred mixture of o-aminophenol (76.3 g, 0.70 mole), 85% o-nitrophenol (40.0 g, 0.24 mole), and 37% aqueous HCl (228 g, 2.31 mole) is heated to 95° under $N_2$ and treated with 2-ethylacrolein (106.0 g, 1.26 mole) over a two hour period. After holding for a one hour period, the reaction is quenched with a total of 425 g of water and the pH is adjusted to 7.0 with 50% aqueous NaOH (160 g, 2.0 mole). The organic layer is separated, cooled, and dried in vacuo at 90° to give the product as a dark brown solid (179.0 g, 67.4% purity).

EXAMPLE 7

Preparation of 3-methyl-8-hydroxyquinoline hydrochloride

A stirred mixture of o-aminophenol (5.18 kg, 47.5 mole), o-nitrophenol (2.91 kg, 20.9 mole), and 37% aqueous HCl (7.27 kg, 73.7 mole) is heated to 85-90° and treated with 2-methylacrolein over a 2 1/4 hour period. When addition is complete, the reaction temperature is held at 90° for one hour then cooled to 50°. Isobutyl alcohol (20.4 kg) and toluene (21.9 kg) are added and the water is removed by azeotropic distillation; the remaining reaction mixture is cooled and filtered. The filter cake is washed with isobutyl alcohol and toluene and dried in vacuo at 60° to give the hydrochloride salt of the product as a bright yellow solid (5.28 kg, 96.5% purity).

## Claims

**1.** A method for the preparation of pyridine-2,3-dicarboxylic acids of formula I

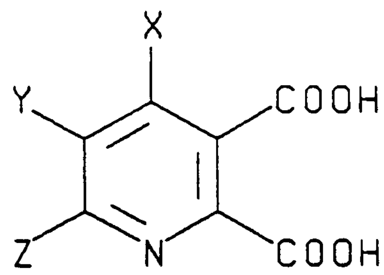

(I)

wherein

X is hydrogen, halogen, or methyl, with the proviso that when Y and Z are taken together to form a ring, and YZ is represented by the structure: $-(CH_2)_n-$, where n is 3 or 4, X is hydrogen;

Y and Z each represent members selected from the group consisting of hydrogen, halogen, $C_1$-$C_6$ alkyl, $C_1$-$C_6$ hydroxyalkyl, $C_1$-$C_6$ haloalkyl, $C_1$-$C_6$ aminoalkyl, $C_1$-$C_6$ sulfonylalkyl, nitro, hydroxy, formyl, carboxy, acyl, amido, amino, $C_1$-$C_4$ alkylamino, diloweralkylamino, $C_1$-$C_4$ alkylsulfonyl, sulfonamido, or phenyl optionally substituted with one $C_1$-$C_4$ alkyl group, $C_1$-$C_4$ alkylsulfonyl group, halogen, hydroxy, or trifluoromethyl group, and when taken together, Y and Z may form a ring in which YZ are represented by the structure: $-(CH_2)_n-$,where n is an integer selected from 3 or 4, provided that X is hydrogen; or

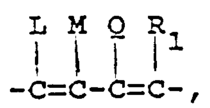

where L, M, Q, and $R_1$ each represent members selected from the group consisting of hydrogen , halogen, $C_1$-$C_4$ alkyl, $C_1$-$C_4$ alkylsulfonyl, $C_1$-$C_4$ haloalkyl, amino $C_1$-$C_4$ alkylamino, diloweralkylamino, and trifluoromethyl, with the proviso that only one of L, M, Q or $R_1$ may represent a substituent other than hydrogen, halogen, or $C_1$-$C_4$ alkyl,

characterized by reacting an 8-hydroxyquinoline compound of formula II

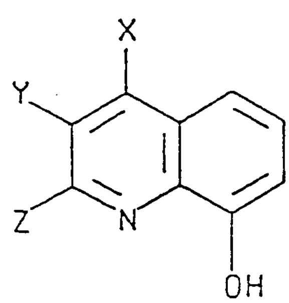

(II)

wherein X, Y, and Z are as described for formula I above with from about 7 to 14 molar equivalents of hydrogen peroxide as a 30%-50% aqueous solution in the presence of 5.5 molar equivalents of aqueous base at 60-100 °C and holding for one hour followed by cooling said reaction mixture to 25-90 °C, adding mineral acid to obtain a pH of about 8-13, then adding about 1.0-2.0 molar equivalents of hypochlorite as a 5%-30% aqueous solution or generated in situ by the addition of chlorine gas and after one to two hours at 65-75 °C, the product dicarboxylic acid may be isolated.

2. A method according to claim 1 wherein 8.0 molar equivalents of hydrogen peroxide and 5.5 molar equivalents of aqueous base are added to a stirred solution of a formula II 8-hydroxyquinoline at a temperature range of from 60-100 °C and is held for one hour after the addition is complete at said temperature range before adding a mineral acid to pH 8-13 and adding 1.0-2.0 molar equivalents of hypochlorite.

3. A method according to claim 2 wherein the pH range of the reaction mixture is 10.5 to 11.5 at the time of the hypochlorite addition.

4. A method according to claim 2 wherein 1.5 molar equivalents of hypochlorite is generated in situ by the direct addition of chlorine gas.

5. A method according to claim 2 wherein 1.5 molar equivalents of hypochlorite is added as a 5%-15% aqueous solution.

6. A method according to claim 2 for the preparation of 5-substituted, 6-substituted, and 5,6-disubstituted pyridine-2,3-dicarboxylic acids from the appropriately substituted 8-hydroxyquinoline precursors or the acid addition salts thereof.

7. A method according to claim 6 for the preparation of 5-ethylpyridine-2,3-dicarboxylic acid from 3-ethyl-8-hydroxyquinoline or the acid addition salt thereof.

8. A method according to claim 6 for the preparation of 5-methylpyridine-2,3-dicarboxylic acid from 3-methyl-8-hydroxyquinoline or the acid addition salt thereof.

9. A method according to claim 6 for the preparation of pyridine-2,3-dicarboxylic acid from 8-hydroxyquinoline or the acid addition salt thereof.

**Patentansprüche**

1. Verfahren zur Herstellung von Pyridin-2,3-dicarbonsäuren der Formel I

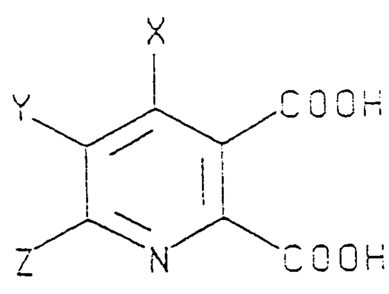

(I)

in der

X Wasserstoff, Halogen oder Methyl ist, mit der Maßgabe, daß wenn Y und Z zusammengenommen werden, um einen Ring zu bilden, und YZ durch die Struktur: $-(CH_2)_n-$ dargestellt wird, worin n 3 oder 4 ist, X Wasserstoff ist;

Y und Z jeweils Mitglieder darstellen, die ausgewählt sind aus der Gruppe, die aus Wasserstoff, Halogen, $C_1$-$C_6$-Alkyl, $C_1$-$C_6$-Hydroxyalkyl, $C_1$-$C_6$-Halogenalkyl, $C_1$-$C_6$-Aminoalkyl, $C_1$-$C_6$-Sulfonylalkyl, Nitro, Hydroxy, Formyl, Carboxy, Acyl, Amido, Amino, $C_1$-$C_4$-Alkylamino, Diniederalkylamino, $C_1$-$C_4$-Alkylsulfonyl, Sulfonamido oder Phenyl, gegebenenfalls mit einer $C_1$-$C_4$-Alkylgruppe, $C_1$-$C_4$-Alkylsulfonylgruppe, Halogen-, Hydroxy- oder Trifluormethylgruppe substituiert, besteht, und Y und Z, wenn sie zusammengenommen werden, einen Ring bilden können, in dem YZ dargestellt wird durch die Struktur: $-(CH_2)_n-$, worin n eine ganze Zahl ist, die aus 3 oder 4 ausgewählt ist, vorausgesetzt, daß X Wasserstoff ist; oder

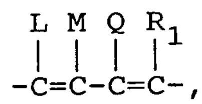

worin L, M, Q und $R_1$ jeweils aus der aus Wasserstoff , Halogen, $C_1$-$C_4$-Alkyl, $C_1$-$C_4$-Alkylsulfonyl, $C_1$-$C_4$-Halogen,Alkylamino, $C_1$-$C_4$-Alkylamino, Diniederalkylamino und Trifluormethyl bestehenden Gruppe ausgewählte Mitglieder darstellen, mit der Maßgabe, daß nur eines von L, M, Q oder $R_1$ einen von Wasserstoff, Halogen oder $C_1$-$C_4$-Alkyl verschiedenen Substituenten darstellen kann,
dadurch gekennzeichnet, daß man eine 8-Hydroxychinolinverbindung der Formel II

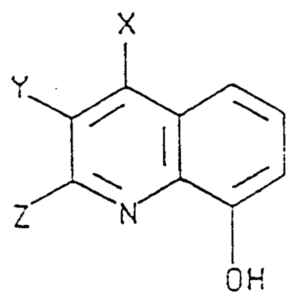

(II)

in der X, Y und Z wie für die Formel I oben beschrieben sind, mit ungefähr 7 bis 14 Moläquivalenten Wasserstoffperoxid als 30-50%iger wäßriger Lösung in Gegenwart von 5,5 Moläquivalenten wäßriger Base bei 60-100 °C umsetzt und 1 Stunde hält, gefolgt vom Abkühlen der Reaktionsmischung auf 25-90 °C, von der Zugabe von Mineralsäure zum Erhalten eines pH von ungefähr 8-13, dann der Zugabe von ungefähr 1,0 - 2,0 Moläquivalenten Hypochlorit als 5-30%iger wäßriger Lösung oder in situ durch die Zugabe von Chlorgas erzeugt, und daß nach einer bis zwei Stunden bei 65-75 °C die Produkt-Dicarbonsäure isoliert werden kann.

**2.** Verfahren nach Anspruch 1, in dem 8,0 Moläquivalente Wasserstoffperoxid und 5,5 Moläquivalente wäßrige Base bei einem Temperaturbereich von 60-100 °C zu einer gerührten Lösung eines 8-Hydroxychinolins der Formel II gegeben werden und nach Beendigung der Zugabe eine Stunde bei dem Temperaturbereich gehalten werden, bevor man eine Mineralsäure bis pH 8-13 zugibt und 1,0 - 2,0 Moläquivalente Hypochlorit zugibt.

**3.** Verfahren nach Anspruch 2, in dem der pH-Bereich der Reaktionsmischung zur Zeit der Hypochlorit-Zugabe 10,5 bis 11,5 ist.

**4.** Verfahren nach Anspruch 2, in dem 1,5 Moläquivalente Hypochlorit in situ durch die direkte Zugabe von Chlorgas erzeugt werden.

**5.** Verfahren nach Anspruch 2, in dem 1,5 Moläquivalente Hypochlorit als 5-15%ige wäßrige Lösung zugegeben werden.

6. Verfahren nach Anspruch 2 zur Herstellung von 5-substituierten, 6-substituierten und 5,6-disubstituierten Pyridin-2,3-dicarbonsäuren aus den geeignet substituierten 8-Hydroxychinolin-Vorläufern oder deren Säureadditionssalzen.

7. Verfahren nach Anspruch 6 zur Herstellung von 5-Ethylpyridin-2,3-dicarbonsäureaus 3-Ethyl-8-hydroxychinolin oder dessen Säureadditionssalz.

8. Verfahren nach Anspruch 6 zur Herstellung von 5-Methylpyridin-2,3-dicarbonsäure aus 3-Methyl-8-hydroxychinolin oder dessen Säureadditionssalz.

9. Verfahren nach Anspruch 6 zur Herstellung von Pyridin-2,3-dicarbonsäure aus 8-Hydroxychinolin oder dessen Säureadditionssalz.

**Revendications**

1. Procédé de préparation d'acides pyridine-2,3-dicarboxyliques de formule I :

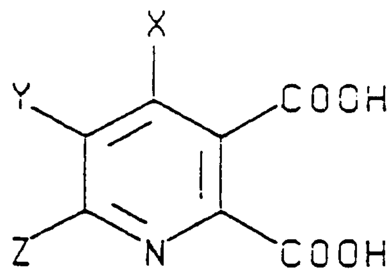

(I)

dans laquelle :

X représente un atome d'hydrogène, d'halogène ou un groupe méthyle, à condition que lorsque Y et Z forment ensemble un cycle, et YZ est représenté par la structure : $-(CH_2)_n-$ dans laquelle n est égal à 3 ou 4, X représente un atome d'hydrogène ;

Y et Z représentent chacun un groupe choisi parmi un atome d'hydrogène, d'halogène, un groupe alkyle en $C_1$-$C_6$, hydroxyalkyle en $C_1$-$C_6$, haloalkyle en $C_1$-$C_6$, aminoalkyle en $C_1$-$C_6$, sulfonylalkyle en $C_1$-$C_6$, nitro, hydroxy, formyle, carboxy, acyle, amido, amino, alkylamino en $C_1$-$C_4$, di(alkyle inférieur)amino, alkylsulfonyle en $C_1$-$C_4$, sulfonamido, ou phényle éventuellement substitué avec un groupe alkyle en $C_1$-$C_4$, alkylsulfonyle en $C_1$-$C_4$, un atome d'halogène, un groupe hydroxy ou un groupe trifluorométhyle, et Y et Z peuvent former ensemble un cycle dans lequel YZ est représenté par la structure $-(CH_2)_n-$ dans laquelle n est un entier égal à 3 ou 4, à condition que X représente un atome d'hydrogène ; ou un groupe

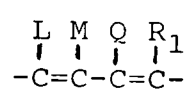

dans lequel L, M, Q et $R_1$ représentent chacun un groupe choisi parmi un atome d'hydrogène, un atome d'halogène, un groupe alkyle en $C_1$-$C_4$, alkylsulfonyle en $C_1$-$C_4$, haloalkyle en $C_1$-$C_4$, amino, alkylamino en $C_1$-$C_4$, di(alkyle inférieur)amino et trifluorométhyle, à condition que seul l'un des groupes L, M, Q et $R_1$ puisse représenter un substituant autre qu'un atome d'hydrogène, d'halogène ou un groupe alkyle en $C_1$-$C_4$,

caractérisé en ce qu'on fait réagir un composé dérivé de 8-hydroxyquinoléine de formule II :

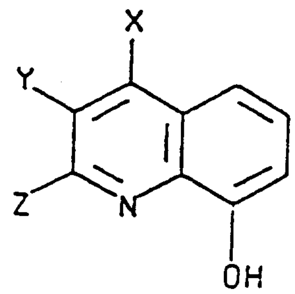

( II )

dans laquelle X, Y et Z sont tels que décrits pour la formule I ci-dessus, avec d'environ 7 à 14 équivalents en moles de peroxyde d'hydrogène sous la forme d'une solution aqueuse à 30 jusqu'à 50 %, en présence de 5,5 équivalents en mole d'une base aqueuse à 60-100 °C, avec maintien pendant une heure puis refroidissement du mélange réactionnel à 25-90 °C, on ajoute un acide minéral pour obtenir un pH d'environ 8 à 13, on ajoute ensuite d'environ 1,0 à 2,0 équivalents en modes d'un hypochlorite sous la forme d'une solution aqueuse à 5 % jusqu'à 30 %, ou généré in situ par addition de chlore gazeux, et après 1 à 2 heures à 65-75 °C, l'acide dicarboxylique produit peut être isolé.

**2.** Procédé selon la revendication 1, dans lequel on ajoute 8,0 équivalents en modes de peroxyde d'hydrogène et 5,5 équivalents en modes d'une base aqueuse, dans une solution agitée de 8-hydroxyquinoléine de formule II à une température de 60 à 100 °C, et on maintient pendant une heure après la fin de l'addition, cette température, avant d'ajouter un acide minéral à un pH de 8 à 13 et de 1,0 à 2,0 équivalents en moles d'un hypochlorite.

**3.** Procédé selon la revendication 2, dans lequel la plage de pH du mélange réactionnel est de 10,5 à 11,5 au moment de l'addition d'un hypochlorite.

**4.** Procédé selon la revendication 2, dans lequel 1,5 équivalents en modes d'hypochlorite sont générés in situ par l'addition directe de chlore gazeux.

**5.** Procédé selon la revendication 2, dans lequel on ajoute 1,5 équivalents en modes d'hypochlorite sous la forme d'une solution aqueuse à 5 % jusqu'à 15 %.

**6.** Procédé selon la revendication 2 pour la préparation d'acides (pyridine 5-substituée, 6-substituée et 5,6-disubstituée)-2,3-dicarboxyliques à partir des précurseurs dérivés de 8-hydroxyquinoléine substituée de manière appropriée, ou de leurs sels d'addition avec un acide.

**7.** Procédé selon la revendication 6 pour la préparation de l'acide 5-éthylpyridine-2,3-dicarboxylique à partir de la 3-éthyl-8-hydroxyquinoléine, ou de son sel d'addition avec un acide.

**8.** Procédé selon la revendication 6 pour la préparation d'acide 5-méthylpyridine-2,3-dicarboxylique à partir de la 3-méthyl-8-hydroxyquinoléine ou de son sel d'addition avec un acide.

**9.** Procédé selon la revendication 6 pour la préparation de l'acide pyridine-2,3-dicarboxylique à partir de la 8-hydroxyquinoléine ou de son sel d'addition avec un acide.